# EUROPEAN PATENT APPLICATION

(11) **EP 1 746 088 A1**
(43) Date of publication of application: **24.01.2007**
(21) Application number: 05015398.0
(22) Date of filing: 15.07.2005
(51) Int. Cl.: C07D 213/04, C07D 277/20, C07D 241/10

(54) **2-Amino-5-aminomethyl-phenol derivatives and agent for coloring keratin fibers comprising these derivatives**

(71) Applicant: Wella Aktiengesellschaft, 64274 Darmstadt (DE)
(72) Inventor: Pasquier, Cécile, 1723 Marly (CH); Tinguely, Eric, 1700 Fribourg (CH); Buclin, Veronique, 1638 Morlon (CH); Braun, Hans-Jürgen, 3182 Ueberstorf (CH)

(57) **Abstract**

The object of the present patent application are new 2-amino-5-aminomethyl-phenol derivatives of general formula (I) and colorants for oxidative dyeing of keratin fibers, particularly human hair, containing at least one 2-amino-5-aminomethyl-phenol derivative of formula (I) as dye component; 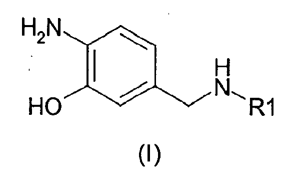 wherein **R1** denotes a five-membered or a six-membered heterocyclic ring, substituted or unsubstituted, or a physiologically tolerated, watersoluble salt thereof, and wherein R1 is not an unsubstituted 3-pyridinyl ring.

## Description

The present invention relates to colorants for oxidative dyeing of keratin fibers, particularly human hair, containing 2-amino-5-aminomethyl-phenol derivatives as dye component and to novel 2-amino-5-aminomethyl-phenol derivatives.

Oxidation dyes have become very important for the coloring of keratin fibers, particularly in the field of hair coloring. In this case, the color is created by reaction of certain developers with certain couplers in the presence of an appropriate oxidant. Suitable developers for this purpose are, in particular, 2,5-diaminotoluene, 2,5-diaminophenylethyl alcohol, p-aminophenol and 1,4-diaminobenzene. Suitable couplers are, for example, resorcinol, 4-chlororesorcinol, 1-naphthol, 3-aminophenol and derivatives of m-phenylenediamine.

Oxidation dyes used for coloring human hair must meet numerous requirements besides producing colorations of a desired intensity. The dyes must be toxicologically and dermatologically harmless, and the resulting hair colors must have good light, permanent wave, acid and rubbing resistance. The hair colors must also remain stable for a period of at least four to six weeks in the absence of light, rubbing and chemicals. In addition, it must be possible, by means of a combination of appropriate developers and couplers, to create a wide range of different color shades.

The adjustment of lighter color shades presents a special problem in terms of uniform dye uptake from the hair roots to the hair tips and in terms of resistance of the colorations to permanent wave treatment. The use of direct yellow-dyeing aromatic nitro dyes together with oxidative hair dye precursors represents a partial solution of the said problem, but the stability of the colorations over a period of several weeks is often unsatisfactory.

To solve the said problem, DE 28 33 989 A1 proposes the use of 2-amino-5-methylphenol as a yellow dyeing oxidative dye in oxidative hair colorants. Although this compound is well suited as shade-adjustment agent for the creation of bright blond shades and gold shades, it does not fully meet requirements. The use of special 2-Amino-5-aminomethyl-phenols in oxidative colorants is also described in WO 02/058654 A1.

We have now found that new 2-amino-5-aminomethyl-phenol derivatives of the general formula (I) provide bright blond to gold color shades, and that the requirements placed on such agents, particularly as regards wash fastness, light fastness and rubbing fastness, are met to an unusually high degree.

The object of the present invention are 2-amino-5-aminomethyl-phenol derivatives of general formula (I) or a physiologically tolerated, water-soluble salt thereof, 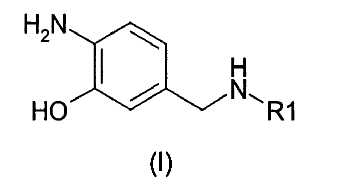 wherein
**R1** denotes a substituted or unsubstituted five-membered or a six-membered heterocyclic ring, and wherein R1 is not an unsubstituted 3-pyridinyl ring.

Suitable 2-amino-5-aminomethyl-phenol derivatives of general of formula (I) are, for example: 2-amino-5-[(2-pyridinylamino)methyl]phenol, 2-amino-5-{[(3-methyl-2-pyridinyl)amino]methyl}phenol, 2-amino-5-{[(4-methyl-2-pyridinyl)amino]methyl}phenol, 2-amino-5-{[(5-methyl-2-pyridinyl)amino]methyl}phenol, 2-amino-5-{[(6-methyl-2-pyridinyl)amino]methyl}phenol, 2-amino-5-{[(4,6-dimethyl-2-pyridinyl)amino]methyl}phenol, 2-amino-5-{[(3-bromo-2-pyridinyl)amino]methyl}phenol, 2-amino-5-{[(4-bromo-2-pyridinyl)amino]methyl}phenol, 2-amino-5-{[(5-bromo-2-pyridinyl)amino]methyl}phenol, 2-amino-5-{[(3,5-dibromo-2-pyridinyl)amino]methyl}phenol, 2-amino-5-{[(3,6-dibromo-2-pyridinyl)amino]methyl}phenol, 2-amino-5-{[(6-bromo-5-methyl-2-pyridinyl)amino]methyl}phenol, 2-amino-5-{[(5-bromo-6-methyl-2-pyridinyl)amino]methyl}phenol, 2-amino-5-{[(3-chloro-2-pyridinyl)amino]methyl}phenol, 2-amino-5-{[(4-chloro-2-pyridinyl)amino]methyl}phenol, 2-amino-5-{[(5-chloro-2-pyridinyl)amino]methyl}phenol, 2-amino-5-{[(3,5-dichloro-2-pyridinyl)amino]methyl}phenol, 2-amino-5-{[(3,6-dichloro-2-pyridinyl)amino]methyl}phenol, 2-amino-5-{[(5-fluoro-2-pyridinyl)amino]methyl}phenol, 6-[(4-amino-3-hydroxybenzyl)amino]nicotinonitrile, 6-[(4-amino-3-hydroxybenzyl)amino]nicotinic acid, 6-[(4-amino-3-hydroxybenzyl)amino]-3-pyridinesulfonic acid, 2-amino-5-[2-chloro(3-pyridinylamino)methyl]phenol, 2-amino-5-[6-chloro(3-pyridinylamino)methyl]phenol, 2-amino-5-[2,6-dichloro(3-pyridinylamino)methyl]phenol, 5-[(4-amino-3-hydroxybenzyl)amino]-2-pyridinecarbonitrile, 2-amino-5-{[(6-methoxy-3-pyridinyl)amino]methyl}phenol, 2-amino-5-[(4-pyridinylamino)methyl]phenol, 2-amino-5-{[(2-methyl-4-pyridinyl)amino]methyl}phenol, 2-amino-5-{[(2-chloro-4-pyridinyl)amino]methyl}phenol, 2-amino-5-[(2-pyrimidinylamino)methyl]phenol, 2-amino-5-{[(4-methyl-2-pyrimidinyl)amino]methyl}phenol, 2-amino-5-{[(4-chloro-2-pyrimidinyl)amino]methyl}phenol, 2-amino-5-{[(4-bromo-2-pyrimidinyl)amino]methyl}phenol, 2-amino-5-{[(4-hydroxy-2-pyrimidinyl)amino]methyl}phenol, 2-amino-5-{[(4-methoxy-2-pyrimidinyl)amino]methyl}phenol, 2-amino-5-{[(5-methyl-2-pyrimidinyl)amino]methyl}phenol, 2-amino-5-{[(5-chloro-2-pyrimidinyl)amino]methyl}phenol, 2-amino-5-{[(5-bromo-2-pyrimidinyl)amino]methyl}phenol, 2-amino-5-{[(5-hydroxy-2-pyrimidinyl)amino]methyl}phenol, 2-amino-5-{[(5-methoxy-2-pyrimidinyl)amino]methyl}phenol, 2-[(4-amino-3-hydroxybenzyl)amino]-5-pyrimidinecarbonitrile, 2-amino-5-{[(4,6-dichloro-2-pyrimidinyl)amino]methyl}phenol, 2-amino-5-{[(4,6-dihydroxy-2-pyrimidinyl)amino]methyl}phenol, 2-amino-5-{[(4,6-dimethoxy-2-pyrimidinyl)amino]methyl}phenol, 2-amino-5-[(2-pyrazinylamino)methyl]phenol, 2-amino-5-{[(3-chloro-2-pyrazinyl)amino]methyl}phenol, 2-amino-5-{[(3-bromo-2-pyrazinyl)amino]methyl}phenol, 2-amino-5-{[(5-chloro-2-pyrazinyl)amino]methyl}phenol, 2-amino-5-{[(5-bromo-2-pyrazinyl)amino]methyl}phenol, 2-amino-5-{[(6-chloro-2-pyrazinyl)amino]methyl}phenol, 2-amino-5-{[(6-bromo-2-pyrazinyl)amino]methyl}phenol, 2-amino-5-[(1,3-thiazol-2-ylamino)methyl]phenol, 2-amino-5-{[(4-methyl-1,3-thiazol-2-yl)amino]methyl}phenol, 2-amino-5-{[(5-methyl-1,3-thiazol-2-yl)amino]methyl}phenol, 2-amino-5-{[(4,5-dimethyl-1,3-thiazol-2-yl)amino]methyl}phenol, 2-amino-5-[(5-isothiazolylamino)methyl]phenol, 2-amino-5-[(5-isoxazolylamino)methyl]phenol, 2-amino-5-[(1*H*-pyrazol-3-ylamino)methyl]phenol, 2-amino-5-[(1*H*-1,2,4-triazol-3-ylamino)methyl]phenol and 2-amino-5-[(1*H*-imidazol-2-ylamino)methyl]phenol or
a physiologically tolerated salt thereof.

Preferred are compounds of formula (I) wherein **R1** is a substituted or unsubstituted 2-pyridinyl group, a substituted or unsubstituted 2-pyrimidinyl group, a substituted or unsubstituted 2-pyrazinyl group, a substituted or unsubstituted 2-thiazolyl group or a substituted 3-pyridinyl group.

Particularly preferred compounds of formula (I) are 2-amino-5-[(2-pyridinylamino)methyl]phenol, 2-amino-5-{[(4-methyl-2-pyridinyl)amino]-methyl}phenol, 2-amino-5-[(2-pyrimidinylamino)methyl]phenol, 2-amino-5-[(2-pyrazinylamino)methyl]phenol and 2-amino-5-[(1,3-thiazol-2-ylamino)-methyl]phenol, 2-amino-5-{[(4-methy)-1,3-thiazol-2-yl)amino]methyl}-phenol, 2-amino-5-{[(5-methyl-1,3-thiazol-2-yl)amino]methyl}phenol, 2-amino-5-{[(4,5-dimethyl-1,3-thiazol-2-yl)amino]methyl}phenol, or a physiologically tolerated salt thereof.

The compounds of formula (I) can be used as free bases or in the form of their physiologically tolerated salts with inorganic or organic acids such as, for example, hydrochloric acid, sulfuric acid, phosphoric acid, acetic acid, propionic acid, lactic acid or citric acid.

The 2-amino-5-aminomethyl-phenol derivatives of formula (I) can be prepared by known synthetic methods, for example by reductive amination of 3-hydroxy-4-nitro-benzaldehyde with a heterocyclic primary amine followed by reduction of the nitro group, or by condensation of 3-hydroxy-4-nitro-benzoic acid with a heterocyclic primary amine, followed by the reduction of the nitro and amide group.

Another object of the present invention are agents for oxidative coloring of keratin fibers, for example wool, furs, feathers or hair, particularly human hair, containing at least one 2-amino-5-aminomethyl-phenol derivative of formula (I).

The colorants of the invention contain the 2-amino-5-aminomethyl-phenol derivative of formula (I) in an amount from about 0.005 to 20 % by weight, an amount from about 0.01 to 5.0 % by weight and particularly from 0.1 to 2.5 % by weight being especially preferred.

Hair coloring compositions of this invention can contain the 2-amino-5-aminomethyl-phenol derivative of formula (I) as a sole colorant, or in combination with known couplers and developers, in order to enlarge the color shade palette.

Thus 2-amino-5-aminomethyl-phenol derivatives of formula (I) provide as a sole colorant intense yellow to yellow-orange color shades. To achieve additional color shades, which are unusually resistant to washing, common oxidative dyes, for example developers or couplers alone or in admixture with one another, can be added.

By use of 2-amino-5-aminomethyl-phenol derivatives of formula (I) in a 1:1 combination with most common coupler or developer components, intense yellow, gold yellow and blond color shades are obtained. The combination with pyrazole derivatives give intense violet color shades.

Suitable couplers include for example, N-((3-dimethylamino)phenyl)urea, 2,6-diaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)-benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)-amino]aniline, 3-[(2-aminoethyl)amino]aniline, 1,3-di(2,4-diaminophenoxy)-propane, di(2,4-diaminophenoxy)methane, 1,3-diamino-2,4-dimethoxybenzene, 2,6-bis(2-hydroxyethyl)amino-toluene, 4-hydroxyindole, 3-dimethylaminophenol, 3-(diethylamino)phenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-amino-phenol, 2-[(3-hydroxyphenyl)-amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]-phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)-amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol-acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 6-hydroxy-benzomorpholine, 6-aminobenzomorpholine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

Suitable developers include, for example, 1,4-diaminobenzene (p-phenylenediamine), 1,4-diamino-2-methylbenzene (p-toluylenediamine), 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 2-(6-2,5-methyl-pyridin-2-yl)-benzene-1,4-diamine, 2-thiazol-2-yl-benzene-1,4-diamino, 1,4-diamino-2-(pyridin-3-yl)benzene, 2,5-diaminobiphenyl, 2,5-diamino-4'-(1-methylethyl)-1,1'-biphenyl, 2,3',5-triamino-1,1'-biphenyl, 2'-chloro-1,1'-biphenyl-2,5-diamine, 3'-fluoro-1,1'-biphenyl-2,5-diamine, 1,4-diamino-2-methoxymethylbenzene, 1,4-diamino-2-aminomethyl-benzene, 3-(3-amino-phenylamino-propenyl)-benzene-1,4-diamine, 2-propenylbenzene-1,4-diamine, 1,4-diamino-2-((phenylamino)methyl)-benzene, 1,4-diamino-2-((ethyl-(2-hydroxyethylyamino)methyl)benzene, 1,4-diamino-2-hydroxymethylbenzene, 1,4-diamino-2-(2-hydroxyethoxy)-benzene, 2-(2-(acetylamino)ethoxy)-1,4-diaminobenzene, 4-(phenyl-amino)aniline, 4-(dimethylamino)aniline, 4-(diethylamino)aniline, 4-(dipropylamino)aniline, 4-[ethyl(2-hydroxyethyl)amino]aniline, 4-[di(2-hydroxyethyl)amino]aniline, 4-[di(2-hydroxyethyl)amino]-2-methylaniline, 4-[(2-methoxyethyl)amino]aniline, 4-[(3-hydroxypropyl)amino]-aniline, 4-[(2,3-dihydroxypropyl)amino]aniline, 4-(((4-aminophenyl)-methyl)amino)aniline, 4-[(4-amino-phenylamino)-methyl]-phenol, 3-((4-amino-phenylamino)methyl)phenol, 1,4-diamino-N-(4-pyrrolidin-1-yl-benzyl)benzene, 1,4-diamino-N-furan-3-ylmethylbenzene, 1,4-diamino-N-thiophen-2-ylmethylbenzene, 1,4-diamino-N-furan-2-ylmethylbenzene, 1,4-diamino-N-thiophen-3-ylmethylbenzene, 1,4-diamino-N-benzylbenzene, 1,4-diamino-2-(1-hydroxyethyl)benzene, 1,4-diamino-2-(2-hydroxyethyl)benzene, 1,4-diamino-2-(1-methylethyl)benzene, 1,3-bis[(4-aminophenyl)(2-hydroxyethyl)amino]-2-propanol, 1,4-bis[(4-Aminophenyl)-amino]-butane, 1,8-bis(2,5-diaminophenoxy)-3,6-dioxaoctane, 2,5-diamino-4'-hydroxy-1,1'-biphenyl, 2,5-diamino-2'-trifluoromethyl-1,1'-biphenyl, 2,4',5-triamino-1,1'-biphenyl, 4-amino-phenol, 4-amino-3-methylphenol, 4-amino-3-(hydroxymethyl)-phenol, 4-amino-3-fluoro-phenol, 4-methylamino-phenol, 4-amino-2-(aminomethyl)phenol, 4-amino-2-(hydroxymethyl)phenol, 4-amino-2-fluorophenol, 4-amino-2-[(2-hydroxyethyl)-amino]methyl-phenol, 4-amino-2-methyl-phenol, 4-amino-2-(methoxymethyl)phenol, 4-amino-2-(2-hydroxyethyl)phenol, bis(5-amino-2-hydroxyphenyl)phenol, 5-amino-salicylic acid, 2,5-diamino-pyridine, 2,4,5,6-tetraamino-pyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 4,5-diamino-1-pentyl-1H-pyrazole, 4,5-diamino-1-(phenylmethyl)-1H-pyrazole, 4,5-diamino-1-((4-methoxyphenyl)methyl-1 H-pyrazole, 2-aminophenol, 2-amino-6-methylphenol, 2-amino-5-methyl-phenol, 4-amino-1,1'-biphenyl-3-ol, 2-amino-5-ethylphenol, 1,2,4-trihydroxybenzene, 2,4-diaminophenol, 1,4-dihydroxybenzene and 2-(((4-aminophenyl)amino)methyl)-1,4-diaminobenzene.

The aforesaid couplers and developers can be used in the colorant of the invention either alone or in admixture with each other.

The total amount of the developer-coupler combination contained in the colorants described here is preferably from about 0.01 to 20 % by weight, an amount from about 0.02 to 10 % by weight and particularly from 0.2 to 6.0 % by weight being particularly preferred. In general, the developers and couplers are used in about equimolar amounts. It is not disadvantageous, however, to use the developer in a certain excess or deficiency, for example in a coupler-developer-ratio of from 1:2 to 1:0.5.

Moreover, the colorant of the invention may also contain other components, for example 6-amino-2-methylphenol, 2-amino-5-methylphenol and 2-amino-5-ethylphenol, as well as common direct dyes, for example triphenylmethane dyes such as [(4'-aminophenyl)-(4'-imino-2",5"-cyclohexadien-1"-ylidene)methyl-2-methylaminobenze monohydrochloride (C.I. 42510) and 4-[(4'-amino-3'-methylphenyl)(4"-imino-3"-methyl-2",5"-cyclohexadient-1"-yl)idine)methyl]-2-methylaminobenzene monohydrochloride (C.I.42520), aromatic nitro dyes, such as 4-(2'-hydroxyethyl)aminonitrotoluene, 2-amino-4,6-dinitrophenol, 2-amino-5-(2'-hydroxyethyl)aminonitrobenzene, 2-chloro-6-(ethylamino)-4-nitrophenol, 4-chloro-N-(2-hydroxyethyl)-2-nitroaniline, 5-chloro-2-hydroxy-4-nitroaniline, 2-amino-4-chloro-6-nitrophenol and 1-[(2'-ureidoethyl)-amino-4-nitrobenzene, furthermore azo dyes such as sodium 6-[(4'-aminophenyl])azo]-5-hydroxynaphthalenesulfonate, and disperse dyes such as, for example 1,4-diaminoanthraquinone and 1,4,5,8-tetraamino-anthraquinone.

The colorants may contain these components in an amount from about 0.1 to 4.0 % by weight.

Naturally, the couplers and developers as well as the other dye components, if they are bases, can also be used in the form of their physiologically tolerated salts with organic or inorganic acids such as, for example, hydrochloric acid or sulfuric acid, or -if they contain aromatic hydroxy groups- in the form of salts with bases, for example as their alkali metal phenoxides.

Moreover, the colorants, if they are intended for coloring hair, can contain other common cosmetic additives, for example antioxidants such as ascorbic acid, thioglycolic acid or sodium sulfite, as well as perfumes, complexing agents, wetting agents, emulsifiers, thickeners and hair-care agents. The form in which the colorant preparations of the invention appear can be, for example, that of a solution, particularly an aqueous or aqueous-alcoholic solution. Particularly preferred preparation forms, however, are creams, gels or emulsions. Their composition consists of a mixture of dye components and the usual additives for such preparations. Conventional additives to solutions, creams, emulsions or gels are, for example, solvents such as water, the lower aliphatic alcohols, for example ethanol, propanol or isopropanol, glycerol or glycols such as 1,2-propylene glycol, moreover wetting agents or emulsifiers from the class of anionic, cationic, amphoteric or nonionic surfactants, such as fatty alcohol sulfates, ethoxylated fatty alcohol sulfates, alkylsulfonates, alkylbenzenesulfonates, alkyltrimethylammonium salts, alkylbetaines, ethoxylated fatty alcohols, ethoxylated nonylphenols, fatty alkanolamides, ethoxylated fatty esters, furthermore thickeners, such as the fatty alcohols, starch or cellulose derivatives, furthermore vaseline, paraffin oil and fatty acids as well as hair-care agents such as cationic resins, lanolin derivatives, cholesterol, pantothenic acid and betaine. The said constituents are employed in amounts normally used for such purposes, for example the wetting agents and emulsifiers in an amount from about 0.5 to 30 % by weight, the thickeners in an amount from about 0.1 to 25 % by weight and the hair-care agents at a concentration of about 0.1 to 5 % by weight.

Depending on the composition, the colorant of the invention can be weakly acidic, neutral or alkaline. In particular, it has a pH from 6.8 to 11.5.

According to the present invention for pH adjustement in the alkaline range the composition may further optionally comprise at least one source of alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonium hydrogen carbonate, ammonia and mixtures thereof or a mixture of ammonia and organic amines (particularly monoethanolamine or triethanolamine). The compositions of the present invention may comprise from about 0.1% to about 10% by weight, preferably from about 0.5% to about 5%, most preferably from about 1% to about 3% of an alkalizing agent, preferably ammonium ions.

For pH adjustment in the acidic range, an inorganic or organic acid, for example phosphoric acid, acetic acid, citric acid or tartaric acid, may be used.

The compositions according to the present invention may comprise at least one source of an oxidizing agent for developing the hair color. Preferred oxidizing agents for use herein are water-soluble peroxygen oxidizing agents. "Water-soluble" as defined herein means that in standard condition at least 0.1 g, preferably 1 g, more preferably 10g of said oxidizing agent can be dissolved in 1 liter of deionized water. The oxidizing agents are valuable for the initial solubilisation and decolourisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the compositions according to the present invention are hydrogen peroxide, percarbonate, persulphates and combinations thereof.

According to the present invention the compositions comprise from about 0.1 % to about 15% by weight, preferably from about 1 % to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably 7.5 to 9.5 more preferably about pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from about 0.1 % to about 15% by weight, preferably from about 1% to about 10% by weight, and most preferably from about 1% to about 8% by weight of a hydrogencarbonate ion and from about 0.1% to about 10% by weight, preferably from about 1% to about 7% by weight, and most preferably from about 2% to about 5% by weight of a source of hydrogen peroxide.

Especially preferred oxidants for developing the hair color are mainly hydrogen peroxide or a compound of addition of hydrogen peroxide to urea, melamine, sodium borate or sodium carbonate, in the form of a 3 to 12%, preferably 6%, aqueous solution, as well as air oxygen. When a 6% hydrogen peroxide solution is used as the oxidant, the weight ratio of hair colorant to oxidant is 5:1 to 2:1, and preferably 1:1. Larger amounts of oxidant are used primarily when the hair colorant contains a higher dye concentration or when stronger hair bleaching is desired at the same time.

According to the present invention the compositions may further comprise a source of radical scavenger. As used herein the term radical scavenger refers to a species that can react with a reactive radical, preferably carbonate radicals, to convert the reactive radical by a series of fast reactions to a less reactive species. Suitable radical scavengers for use herein include compounds according to the general formula (I):

R¹-Y-C(H)(R³)-R⁴-(C(H)(R⁵)-Y-R⁶)ₙ (I)

wherein Y is NR², O, or S, preferably NR², n is 0 to 2, and wherein R⁴ is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein R⁴ can be connected to R³ or R⁵ to create a 5, 6 or 7 membered ring; and wherein R¹, R², R³, R⁵, and R⁶ are monovalent and are selected independently from: (a), (b) and (c) described herein above, or H.
Preferably, R⁴ is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably R⁴ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.
Preferably, the R⁴ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; and most preferred is O.
Preferably, R¹, R², R³, R⁵, and R⁶ are selected independently from any of the systems defined for R⁴ above, and H.
In alternative embodiments, any of R¹, R², R³, R⁴, R⁵, and R⁶ groups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, mono-, poly-, or per-fluoro alkyl systems; said systems of (i), (ii) and (iii) comprising from 1 to 10 carbon atoms and 0 to 5 heteroatoms selected from O, S, N, P, and Si; (b) the group of S-linked monovalent substituents consisting of SA¹, SCN, SO₂A¹, SO₃A¹, SSA¹, SOA¹, SO₂NA¹A², SNA¹A², and SONA¹A²; (c) the group of O-linked monovalent substituents consisting of OA¹, OCN and ONA¹A²; (d) the group of N-linked monovalent substituents consisting of NA¹A², (NA¹A²A³)⁺, NC, NA¹OA², NA¹SA², NCO, NCS, NO₂, N=NA¹, N=NOA¹, NA¹CN, NA¹NA²A³; (e) the group of monovalent substituents consisting of COOA¹, CON₃, CONA¹₂, CONA¹COA², C(=NA¹)NA¹A², CHO, CHS, CN, NC, and X; and (f) the group consisting fluoroalkyl monovalent substituents consisting of mono-, poly-, or per-fluoro alkyl systems comprising from 1 to 12 carbon atoms and 0 to 4 heteroatoms.
For the groups (b) to (e), described above, A¹, A², and A³ are monovalent and are independently selected from: (1) H, (2) substituted or unsubstituted, straight or branched, alkyl, mono- or poly-unsaturated alkyl, heteroalkyl, aliphatic, heteroaliphatic, or heteroolefinic systems, (3) substituted or unsubstituted, mono- or poly-cyclic aliphatic, aryl, or heterocyclic systems, or (4) substituted or unsubstituted, mono-, poly-, or per-fluoro alkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.
Preferred substituents for use herein include those having a Hammett Sigma Para (σₚ) Value from -0.65 to +0.75, preferably from -0.4 to +0.5.

Hammett Sigma Values are described in Advanced Organic Chemistry ― Reactions, Mechanisms and Structure (Jerry March, 5th ed. (2001) at pages 368-375).
Alternative suitable radical scavengers for use herein are compounds according to the general formula (II) : 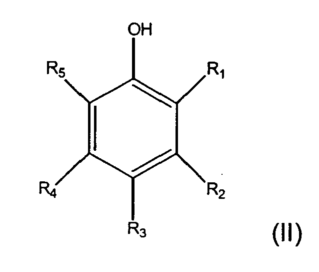 wherein R₁, R₂, R₃, R₄, and R₅ are each independently selected from H, COO⁻M⁺, Cl, Br, SO₃⁻M⁺, NO₂, OCH₃, OH or a C¹ to C¹⁰ primary or secondary alkyl and M is either H or alkali metal. Preferably, the above-described radical scavengers have a pKa of more than 8.5 to ensure protonation of the hydroxy goup.
Other suitable radical scavengers for use herein include those selected from group (III) benzylamine, imidazole, di-tert-butylhydroxytoluene, hydroquinone, guanine, pyrazine, piperidine, morpholine, methylmorpholine, 2methyoxyethylamine, and mixtures thereof.
Preferred radical scavengers according to the present invention are selected from the classes of alkanolamines, amino sugars, amino acids, esters of amino acids and mixtures thereof. Particularly preferred compounds are: monoethanolamine, 3-amino-1-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.
The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1 % to about 10% by weight, preferably from about 1% to about 7% by weight of radical scavenger.
The radical scavenger is also preferably selected such that it is not an identical species as the alkalizing agent. According to one embodiment of the present invention the radical scavenger may be formed insitu in the hair dyeing compositions prior to application to the hair fibres.

To use the afore-described colorants for oxidative dyeing of hair, said colorants are mixed with an oxidant immediately before use, and the mixture is applied to hair in an amount sufficient for hair treatment which, depending on hair fullness, is generally from about 60 to 200 grams.

Suitable oxidants for developing the hair color are mainly hydrogen peroxide or a compound of addition of hydrogen peroxide to urea, melamine, sodium borate or sodium carbonate, in the form of a 3 to 12% by weight, preferably 6% by weight, aqueous solution, as well as air oxygen. When a 6% hydrogen peroxide solution is used as the oxidant, the weight ratio of hair colorant to oxidant is 5:1 to 2:1, and preferably 1:1. Larger amounts of oxidant are used primarily when the hair colorant contains a higher dye concentration or when stronger hair bleaching is desired at the same time.

The mixture is allowed to act on the hair at 15 to 50 °C for about 10 to 45 minutes and preferably for 30 minutes. The hair is then rinsed with water and dried. Optionally, this rinse can be followed with a shampoo wash, optionally followed by rinsing with a weak organic acid, for example citric or tartaric acid. The hair is then dried.

The colorant of the invention containing a 2-amino-5-aminomethyl-phenol derivatives of formula (I) affords colorations of excellent color fastness, particularly in terms of wash fastness, light fastness and rubbing fastness. As regards the coloring properties, the colorants of the invention, provide without further coupler or developer intense yellow to yellow-orange color shades. Depending on the type and composition of the dye components, the colorants of the invention can provide a wide range of different color shades ranging from blond, gold yellow, brown, purple, violet to blue and black shades.

The following examples illustrate the object of the invention in more detail without limiting its scope.

### EXAMPLES

### Example 1: Synthesis of 2-amino-5-[(2-pyridinylamino)methyl]phenol

### 1.1 Synthesis of 2-nitro-5-[(2-pyridinylamino)methyl]phenol

20.0 g (0.12 mole) of 3-hydroxy-4-nitro-benzaldehyde and 14.7 g (0.156 mole) of 2-aminopyridine were dissolved in 450 ml of 1,2-dichloroethane and the resulting mixture was heated to reflux for 6 hours using a water separator. The reaction mixture was then allowed to cool to room temperature. In small portions 10 g (0.156 mol) of acetic acid and 40.7 g (0.192 mol) of sodium triacetoxyborohydride were added to that mixture. The reaction mixture was stirred at room temperature overnight. It was then poured into 450 ml 10 % NaHCO3. The organic layer was separated, and the aqueous phase was extracted with ethylacetate. The combined extracts were washed with water, evaporated, and the residue was purified over silicagel with ethylacetate to yield a brown compound, which was recristallized in ethylacetate/heptane to yield 19.3 g (65 % of theory) of a brown powder.
Mp: 115-117°C
API-ES MS: 268 [M⁺+Na].

### 1.2 Synthesis of 2-amino-5-[(2-pryidinylamino)methyl]phenol

At room temperature a solution of 19 g (0.077 mol) of 2-nitro-5-[(2-pyridinylamino)methyl]-phenol in 200 ml of tetrahydrofurane was added dropwise to 9.7 g (0.19 mol) of hydrazine hydrate and 1.9 g 10% Pd/C in 200 ml ethanol. The reaction mixture was stirred at room temperature overnight, filtered through a plug of celite and the residue was washed with ethanol. The filtrate was evaporated and the residue was co-evaporated twice with ethanol (to remove the excess of hydrazine). The residue was suspended in 120 ml of ethanol/ethyl acetate (5:1) and 200 ml of ethylether were added. The precipitate was filtered off and dried, to yield 11 g (66 % of theory) of 2-amino-5-[(2-pyridinylamino)methyl]phenol as a beige powder.
Mp: 136-138°C
¹H-NMR (300 MHz, DMSO): δ = 8.89 (br s, 1H, OH); 7.94 (d, J=4.5, 1H, H(6)-pyridinyl); 7.32 (t, J=7.8, 1 H, H(4)-pyridinyl); 6.74 (t, J=5.7, 1H, NH-CH2); 6.64 (s, 1H, H(6); 6.48 (s, 2H, H(3) and H(4); 6.44 (d, J=7.8, 1 H, H(3)-pyridinyl); 6.42 (d, J=4.5, 1H, H(5)-pyridinyl); 4.37 (br s, 2H, NH2); 4.22 (d, J=5.7, 2H, CH2-NH).
API-ES MS: 238 [M⁺+Na] (100); 216 [M⁺+1] (42).

| CHN-Analysis: (C₁₂H₁₃N₃O): | | | |
|---|---|---|---|
| | % C | % H | % N |
| Calculated: | 66.96 | 6.09 | 19.52 |
| Found: | 66.68 | 6.24 | 19.63 |

### Example 2: Synthesis of 2-amino-5-aminomethyl-phenol derivatives of formula (I) (General method of synthesis)

### 2.1 Synthesis of 2-nitro-5-aminomethyl-phenol derivatives

1 g (6 mmole) of 3-hydroxy-4-nitro-benzaldehyde and 6 mmole of the corresponding amine were dissolved in 20 mL of 1,2-dichloroethane in a screw cap tube under argon. To that mixture were added 7 ml (0.007 mol) of 1 M acetic acid in 1,2- dichloroethane and the resulting mixture was heated to reflux for 3 hours. It was then allowed to cool to room temperature. To that mixture were added 2 g (0.0096 mol) of sodium triacetoxyborohydride and the reaction mixture was stirred at room temperature overnight. It was then poured into 40 ml saturated NaHCO3. The organic layer was separated, and the aqueous phase was extracted with ethylacetate. The combined extracts were washed with water, evaporated, and the residue was purified by column chromatography over silica gel. The product was obtained in a yield of 35 % to 60 % of theory.

### 2.2 Synthesis of 2-amino-5-(aminomethyl)phenol-derivatives

To a solution of 0.3 g of 2-nitro-5-aminomethyl-phenol derivative (as described under 2.1) in 20 ml of a mixture of ethanol/tetrahydrofurane (4:1 to 1:1, depending on the solubility of the nitro derivative) 0.15 ml (3 mmol) of hydrazine hydrate and 0.03 g of 10 % Pd/C were added. The reaction mixture was stirred at room temperature for a few hours, until the reduction was finished (according to TLC). It was then filtered through a plug of celite and the residue was washed with ethanol. The filtrate was evaporated, and the residue was co-evaporated twice with ethanol. The oily residue was triturated with ethylether and the precipitate was filtered off and dried.
a) 2-amino-5-{[(4-methyl-2-pyridinyl)amino]methyl}phenol
   Amine used: 2-amino-4-methylpyridine
   Yield: 0.14 g (50% of the theory)
   API-ES MS: 230(M⁺+1)
   ¹H-NMR (300 MHz, DMSO): δ = 8.90 (br s, 1 H, OH); 7.80 (d, J=5.1, 1 H, H(6)-pyridinyl); 6.63 (s, 1H, H(6); 6.61 (t, J=6.0, 1 H, NH-CH2); 6.50 (s, 2H, H(3) and H(4); 6.30 (d, J=5.1, 1H, H(5)-pyridinyl); 6.26 (s, 1H, H(3)-pyridinyl); 4.35 (br s, 2H, NH2); 4.22 (d, J=6.0, 2H, CH2-NH); 2.11 (s, 3H, CH3).
b) 2-amino-5-[(2-pyrimidinylamino)methyl]phenol
   Amine used: 2-aminopyrimidine
   Yield: 0.25 g (95 % of theory)
   API-ES MS: 247 (M⁺ +1)
   ¹H-NMR (300 MHz, DMSO): δ = 8.88 (br s, 1 H, OH); 8.24 (d, J=4.8, 2H, H(6) and H(4)-pyrimidinyl); 7.42 (t, J=6.3, 1 H, NH-CH2); 6.62 (s, 1H, H(6); 6.53 (t, J=4.8, 1 H, H(5)-pyrimidinyl); 6.49 (s, 2H, H(3) and H(4); 4.35 (br s, 2H, NH2); 4.28 (d, J=6.3, 2H, CH2-NH).
c) 2-amino-5-[(2-pyrazinylamino)methyl]phenol
   Amine used: 2-aminopyrazine
   Yield: 0.22 g (85 % of theory)
   API-ES MS: 239 (M⁺ +Na)
   ¹H-NMR (300 MHz, DMSO): δ = 8.96 (br s, 1 H, OH); 7.91 (d, J=2.4, 2H, H(6) and H(5)-pyrazinyl); 7.62 (d, J=2.4, 1H, H(3)-pyrazinyl); 7.33 (t, J=5.1, 1H, NH-CH2); 6.63 (s, 1H, H(6); 6.52 (s, 2H, H(3) and H(4); 4.40 (br s, 2H, NH2); 4.24 (d, J=5.7, 2H, CH2-NH).
d) 2-amino-5-{[(2-chloro-3-pyridinyl)amino]methyl}phenol
   Amine used: 3-amino-2-chloropyridine
   Yield: 1.0 g (60% of the theoretical)
   API-ES MS: 302 (M⁺ + Na)
   ¹H-NMR (300 MHz, DMSO): δ = 8.94 (br s, 1H, OH); 7.54 (d, J=4.5, 1 H, H(6)-pyridinyl); 7.08 (dd, J=4.1, J=8.1, 1H, H(5)-pyridinyl); 6.85 (d, J=8.1, 1H, H(4)-pyridinyl); 6.62 (s, 1H, H(6)); 6.54 (d, J=9.1, 1 H, H(3) or H(4)); 6.52 (d, J=9.1, 1H, H(3) or H(4)); 6.16 (t, J=5.7, 1 H, NH-CH2); 4.39 (br s, 2H, NH2); 4.19 (d, J=5.7, 2H, CH2-NH).
e) 2-amino-5-[(1,3-thiazol-2-ylamino)methyl]phenol
   Amine used: 2-amino-thiazole
   Yield: 0.24 g (89% of the theoretical)
   API-ES MS: 244 (M⁺ +Na)
   ¹H-NMR (300 MHz, DMSO): δ = 8.92 (br s, 1 H, OH); 7.82 (t, J=5.4, 1 H, NH-CH2); 6.99 (d, J=3.3, 1 H, H(4)-thiazolyl); 6.65 (s, 1H, H(6); 6.56 (d, J=3.3, 1 H, H(5)-thiazolyl); 6.52 (s, 2H, H(3) and H(4); 4.42 (br s, 2H, NH2); 4.19 (d, J=5.4, 2H, CH2-NH).
f) 2-amino-5-{[(4-methyl-1,3-thiazol-2-yl)amino]methyl}phenol
   Amine used: 2-amino-5-methyl-thiazole
   Yield: 0.15 g (53% of the theoretical)
   API-ES MS: 258 (M⁺ +Na)
   ¹H-NMR (300 MHz, DMSO): δ = 8.95 (br s, 1 H, OH); 7.69 (t, J=5.4, 1 H, NH-CH2); 6.64 (s, 1H, H(6)); 6.51 (s, 2H, H(3) and H(4); 6.10 (s, 1H, H(5)-thiazolyl); 4.41 (br s, 2H, NH2); 4.16 (d, J=5.4, 2H, CH2-NH); 2.08 (s, 3H, CH3).
g) 2-amino-5-{[(5-methyl-1,3-thiazol-2-yl)amino]methyl}phenol
   Amine used: 2-amino-4-methyl-thiazole
   Yield: 0.24 g (91 % of the theoretical)
   API-ES MS: 258 (M⁺ +Na)
   ¹H-NMR (300 MHz, DMSO): δ = 8.95 (br s, 1 H, OH); 7.57 (t, J=5.4, 1 H, NH-CH2); 6.63 (s, 2H, H(4)-thiazolyl and H(6)); 6.50 (s, 2H, H(3) and H(4); 4.41 (br s, 2H, NH2); 4.13 (d, J=5.4, 2H, CH2-NH); 2.18 (s, 3H, CH3).
h) 2-amino-5-{[(4,5-dimethyl-1,3-thiazol-2-yl)amino]methyl}phenol
   Amine used: 2-amino-4,5-dimethyl-thiazole
   Yield: 0.22 g (81 % of the theoretical)
   API-ES MS: 272 (M⁺ +Na)
   ¹H-NMR (300 MHz, DMSO): δ = 8.95 (br s, 1 H, OH); 7.44 (t, J=5.4, 1 H, NH-CH2); 6.63 (s, 1H, H(6)); 6.50 (s, 2H, H(3) and H(4); 4.40 (br s, 2H, NH2); 4.11 (d, J=5.4, 2H, CH2-NH); 2.09 (s, 3H, CH3); 1.98 (s, 3H, CH3).

### Examples 3 to 16: Hair Colorants (1:1 combination)

| | |
|---|---|
| 0.00125 mole | of 2-amino-5-[(2-pyridinylamino)methyl]-phenol (example 1.2) |
| 0.00125 mole | of developer or coupler as described in table 1 |
| 10.0 g | of sodium laurylethersulfate (28% aqueous solution) |
| 9.1 g | of ammonia (25% aqueous solution) |
| 7.85 g | of ethanol |
| 0.3 g | of ascorbic acid |
| 0.3 g | of EDTA |
| 0.4 g | of sodium sulfite |
| to 100.0 g | water |

Immediately before use, 30 g of the above mentioned colorant solution was mixed with 30 g of 6% hydrogen peroxide solution. The mixture was then applied to bleached hair. After an exposure time of 30 min at 40 °C, the hair was rinsed with water, washed with a commercial shampoo and dried. The resulting color shades are shown in Table 1.

**Table 1: Hair colorant (1:1 combination)**

| **Example No.** | **dye of formula (I)** | **developer/coupler** | **color result** |
|---|---|---|---|
| **3** | 2-amino-5-[(2-pyridinyl-amino)methyl]-phenol (as in example 1.2) | 2-amino-5-[(2-pyridinyl-amino)methyl]-phenol (as in example 1.2) | gold yellow |
| **4** | as in example 1.2 | 1,4-diamino-2-methylbenzene sulfate | blond |
| **5** | as in example 1.2 | 1,4-diamino-2-(1-hydroxyethyl)benzene sulfate | blond |
| **6** | as in example 1.2 | 4-aminophenol | gold yellow |
| **7** | as in example 1.2 | 4-amino-3-methylphenol | gold yellow |
| **8** | as in example 1.2 | 5-amino-2-methylphenol | gold yellow-orange |
| **9** | as in example 1.2 | 3-aminophenol | gold yellow |
| **10** | as in example 1.2 | 1,3-dihydroxybenzene | gold yellow |
| **11** | as in example 1.2 | 1,3-dihydroxy-2-methyl-benzene | blond-yellow |
| **12** | as in example 1.2 | 2,4-diamino-1-(2-hydroxyethoxy)benzene sulfate | blond-yellow |
| **13** | as in example 1.2 | 2-amino-4-[(2-hydroxyethyl)amino]-anisole-sulfate | blond-yellow |
| **14** | as in example 1.2 | 1-naphthol | gold yellow-orange |
| **15** | as in example 1.2 | 4-di[(2-hydroxyethyl)-amino]aniline sulfate | blond-green |
| **16** | as in example 1.2 | 4,5-diamino-1-(2-hydroxyethyl)-1H-pyrazole sulfate | violet |

### Examples 17 to 106 : Hair Colorants (1:1 combination)

| | |
|---|---|
| 0.00125 mole | of 2-amino-5-aminomethylphenol of formula (I) [A2 to A9 according to table 2] |
| 0.00125 mole | of developer or coupler according to table 3 |
| 10.0 g | of sodium laurylethersulfate (28% aqueous solution) |
| 9.1 g | of ammonia (25% aqueous solution) |
| 7.85 g | of ethanol |
| 0.3 g | of ascorbic acid |
| 0.3 g | of EDTA |
| 0.4 g | of sodium sulfite |
| to 100.0 g | water |

Immediately before use, 30 g of the above mentioned colorant solution was mixed with 30 g of 6% hydrogen peroxide solution. The mixture was then applied to bleached hair. After an exposure time of 30 min at 40 °C, the hair was rinsed with water, washed with a commercial shampoo and dried. The resulting color shades are shown in Table 3.

**Table 2:**

| 2-amino-5-(aminomethyl)phenol-derivatives of formula (I) | |
|---|---|
| **A1** | 2-amino-5-[(2-pyridinylamino)methyl]phenol (example 1.2) |
| **A2** | 2-amino-5-{[(4-methyl-2-pyridinyl)amino]methyl}phenol (example 2.2a) |
| **A3** | 2-amino-5-[(2-pyrimidinylamino)methyl]phenol (example 2.2b) |
| **A4** | 2-amino-5-[(2-pyrazinylamino)methyl]phenol (example 2.2c) |
| **A5** | 2-amino-5-{[(2-chloro-3-pyridinyl)amino]methyl}phenol (example 2.2d) |
| **A6** | 2-amino-5-[(1,3-thiazol-2-ylamino)methyl]phenol (example 2.2e) |
| **A7** | 2-amino-5-{[(4-methyl-1,3-thiazol-2-yl)amino]methyl}phenol (example 2.2f) |
| **A8** | 2-amino-5-{[(5-methyl-1,3-thiazol-2-yl)amino]methyl}phenol (example 2.2g) |
| **A9** | 2-amino-5-{[(4,5-dimethyl-1,3-thiazol-2-yl)amino]methyl}phenol (example 2.2h) |

**Table 3: Hair colorants (1:1 combination)**

| **Example No.** | **dye of formula (I)** | **developer/coupler** | **color result** |
|---|---|---|---|
| **17** | A2 | A2 | gold yellow |
| **18** | A3 | A3 | yellow-orange |
| **19** | A4 | A4 | yellow-orange |
| **20** | A5 | A5 | gold yellow |
| **21** | A6 | A6 | yellow-orange |
| **22** | A7 | A7 | yellow-orange |
| **23** | A8 | A8 | yellow-orange |
| **24** | A9 | A9 | gold yellow |
| **25** | A2 | 1,4-diamino-2-methylbenzene sulfate | blond |
| **26** | A3 | 1,4-diamino-2-methylbenzene sulfate | blond |
| **27** | A4 | 1,4-diamino-2-methylbenzene sulfate | blond |
| **28** | A5 | 1,4-diamino-2-methylbenzene sulfate | middle blond |
| **29** | A6 | 1,4-diamino-2-methylbenzene sulfate | middle blond |
| **30** | A7 | 1,4-diamino-2-methylbenzene sulfate | middle blond |
| **31** | A8 | 1,4-diamino-2-methylbenzene sulfate | middle blond |
| **32** | A9 | 1,4-diamino-2-methylbenzene sulfate | middle blond |
| **33** | A2 | 1,4-diamino-2-(1-hydroxyethyl)-benzene sulfate | blond |
| **34** | A3 | 1,4-diamino-2-(1-hydroxyethyl)-benzene sulfate | blond |
| **35** | A4 | 1,4-diamino-2-(1-hydroxyethyl)-benzene sulfate | blond |
| **36** | A5 | 1,4-diamino-2-(1-hydroxyethyl)-benzene sulfate | middle blond |
| **37** | A6 | 1,4-diamino-2-(1-hydroxyethyl)-benzene sulfate | middle blond |
| **38** | A7 | 1,4-diamino-2-(1-hydroxyethyl)-benzene sulfate | middle blond |
| **39** | A8 | 1,4-diamino-2-(1-hydroxyethyl)-benzene sulfate | middle blond |
| **40** | A9 | 1,4-diamino-2-(1-hydroxyethyl)-benzene sulfate | middle blond |
| **41** | A2 | 4-aminophenol | yellow-apricot |
| **42** | A3 | 4-aminophenol | gold yellow |
| **43** | A4 | 4-aminophenol | gold yellow |
| **44** | A5 | 4-aminophenol | gold yellow |
| **45** | A6 | 4-aminophenol | gold yellow |
| **46** | A7 | 4-aminophenol | gold yellow |
| **47** | A8 | 4-aminophenol | gold yellow |
| **48** | A9 | 4-aminophenol | gold yellow |
| **49** | A2 | 4-amino-3-methylphenol | gold yellow |
| **50** | A3 | 4-amino-3-methylphenol | gold yellow |
| **51** | A4 | 4-amino-3-methylphenol | gold yellow |
| **52** | A5 | 4-amino-3-methylphenol | gold yellow |
| **53** | A6 | 4-amino-3-methylphenol | gold yellow |
| **54** | A7 | 4-amino-3-methylphenol | gold yellow |
| **55** | A8 | 4-amino-3-methylphenol | gold yellow |
| **56** | A9 | 4-amino-3-methylphenol | gold yellow |
| **57** | A2 | 5-amino-2-methylphenol | gold yellow orange |
| **58** | A3 | 5-amino-2-methylphenol | gold yellow orange |
| **59** | A4 | 5-amino-2-methylphenol | gold yellow orange |
| **60** | A5 | 5-amino-2-methylphenol | yellow orange |
| **61** | A6 | 5-amino-2-methylphenol | gold yellow orange |
| **62** | A7 | 5-amino-2-methylphenol | gold yellow orange |
| **63** | A8 | 5-amino-2-methylphenol | gold yellow orange |
| **64** | A9 | 5-amino-2-methylphenol | gold yellow orange |
| **65** | A2 | 3-aminophenol | gold yellow |
| **66** | A3 | 3-aminophenol | yellow |
| **67** | A4 | 3-aminophenol | gold yellow |
| **68** | A6 | 3-aminophenol | gold yellow |
| **69** | A7 | 3-aminophenol | gold yellow |
| **70** | A8 | 3-aminophenol | gold yellow |
| **71** | A9 | 3-aminophenol | gold yellow |
| **72** | A2 | 1,3-dihydroxybenzene | gold yellow |
| **73** | A3 | 1,3-dihydroxybenzene | gold yellow |
| **74** | A4 | 1,3-dihydroxybenzene | gold yellow |
| **75** | A5 | 1,3-dihydroxybenzene | yellow |
| **76** | A6 | 1,3-dihydroxybenzene | gold yellow |
| **77** | A7 | 1,3-dihydroxybenzene | gold yellow |
| **78** | A8 | 1,3-dihydroxybenzene | gold yellow |
| **79** | A9 | 1,3-dihydroxybenzene | gold yellow |
| **80** | A2 | 2,4-diamino-1-(2-hydroxy-ethoxy)benzene sulfate | blond-yellow |
| **81** | A3 | 2,4-diamino-1-(2-hydroxy-ethoxy)benzene sulfate | blond-yellow |
| **82** | A4 | 2,4-diamino-1-(2-hydroxy-ethoxy)benzene sulfate | blond-yellow |
| **83** | A6 | 2,4-diamino-1-(2-hydroxy-ethoxy)benzene sulfate | blond-yellow |
| **84** | A2 | 2-amino-4-[(2-hydroxyethyl)-amino]anisole-sulfate | blond-yellow |
| **85** | A3 | 2-amino-4-[(2-hydroxyethyl)-amino]anisole-sulfate | blond-yellow |
| **86** | A4 | 2-amino-4-[(2-hydroxyethyl)-amino]anisole-sulfate | blond-yellow |
| **87** | A6 | 2-amino-4-[(2-hydroxyethyl)-amino]anisole-sulfate | blond-yellow |
| **88** | A7 | 2-amino-4-[(2-hydroxyethyl)-amino]anisole-sulfate | blond-yellow |
| **89** | A8 | 2-amino-4-[(2-hydroxyethyl)-amino]anisole-sulfate | blond-yellow |
| **90** | A9 | 2-amino-4-[(2-hydroxyethyl)-amino]anisole-sulfate | blond-yellow |
| **91** | A2 | 4-di[(2-hydroxyethyl)-amino]aniline sulfate | blond green |
| **92** | A3 | 4-di[(2-hydroxyethyl)-amino]aniline sulfate | blond green |
| **93** | A4 | 4-di[(2-hydroxyethyl)-amino]aniline sulfate | blond green |
| **94** | A5 | 4-di[(2-hydroxyethyl)-amino]aniline sulfate | dark blond green |
| **95** | A6 | 4-di[(2-hydroxyethyl)-amino]aniline sulfate | dark blond green |
| **96** | A7 | 4-di[(2-hydroxyethyl)-amino]aniline sulfate | dark blond green |
| **97** | A8 | 4-di[(2-hydroxyethyl)-amino]aniline sulfate | dark blond green |
| **98** | A9 | 4-di[(2-hydroxyethyl)-amino]aniline sulfate | dark blond green |
| **99** | A2 | 4,5-diamino-1-(2-hydroxy-ethyl)-1 H-pyrazole sulfate | violet |
| **100** | A3 | 4,5-diamino-1-(2-hydroxy-ethyl)-1H-pyrazole sulfate | violet |
| **101** | A4 | 4,5-diamino-1-(2-hydroxy-ethyl)-1H-pyrazole sulfate | violet |
| **102** | A5 | 4,5-diamino-1-(2-hydroxy-ethyl)-1H-pyrazole sulfate | violet |
| **103** | A6 | 4,5-diamino-1-(2-hydroxy-ethyl)-1H-pyrazole sulfate | violet |
| **104** | A7 | 4,5-diamino-1-(2-hydroxy-ethyl)-1H-pyrazole sulfate | violet |
| **105** | A8 | 4,5-diamino-1-(2-hydroxy-ethyl)-1H-pyrazole sulfate | violet |
| **106** | A9 | 4,5-diamino-1-(2-hydroxy-ethyl)-1H-pyrazole sulfate | violet |

### Examples 107 and 108: Hair Colorants (cream formulation)

| | |
|---|---|
| 0.00125 mole | of 2-amino-5-[(2-pyridinylamino)methyl]phenol (example 1.2) |
| 0.00125 mole | of developer or coupler according to table 4 |
| 15.0 g | of cetylalcohol |
| 3.5 g | of sodium laurylethersulfate (28% aqueous solution) |
| 3.0 g | of ammonia (22% aqueous solution) |
| 0.3 g | of sodium sulfite |
| to 100 g | water |

Immediately before use, 30 g of the above mentioned colorant solution were mixed with 30 g of a 6% hydrogen peroxide solution. The mixture was then applied to bleached hair. After an exposure time of 30 min at 40 °C, the hair was rinsed with water, washed with a commercial shampoo and dried. The resulting color shades are shown in Table 4.

**Table 4: Hair colorant (cream formulation)**

| **Example No.** | **dye of formula (I)** | **developer/coupler** | **color result** |
|---|---|---|---|
| **107** | 2-amino-5-[(2-pyridinyl-amino)methyl] phenol (A1) | 1,4-diamino-2-methylbenzene sulfate | blond |
| **108** | 2-amino-5-[(2-pyridinyl-amino)methyl] phenol (A1) | 5-amino-2-methylphenol | gold yellow orange |

### Examples 109 to 138 : Hair Colorants (multi-combinations)

| | |
|---|---|
| X g | 2-amino-5-[(2-pyridinylamino)methyl] phenol (A1) |
| U g | of developer according to table 5 |
| Y g | of coupler according to table 6 |
| Z g | of direct dye according to table 7 |
| W g | of dye according to table 8 |
| 10.00 g | of sodium laurylethersulfate (28% aqueous solution) |
| 9.10 g | of ammonia (25% aqueous solution) |
| 7.85 g | of ethanol |
| 0.30 g | of ascorbic acid |
| 0.30 g | of EDTA |
| 0.40 g | of sodium sulfite |
| to 100.00 g | water |

Immediately before use, 30 g of the above mentioned colorant solution was mixed with 30 g of a 6 % hydrogen peroxide solution. The mixture was then applied to bleached hair. After an exposure time of 30 min at 40 °C, the hair was rinsed with water, washed with a commercial shampoo and dried. The resulting color shades are shown in table 9.

**Table 5:**

| **Developers** | |
|---|---|
| **E8** | 1,4-diaminobenzene |
| **E9** | 1,4-diamino-2-(1-hydroxyethyl)benzene sulfate |
| **E10** | 4-amino-3-methylphenol |
| **E11** | 4-amino-2-(aminomethyl)phenol dihydrochloride |
| **E12** | 4-aminophenol |
| **E13** | 4-di[(2-hydroxyethyl)amino]aniline sulfate |
| **E14** | 4,5-diamino-1-(2-hydroxyethyl)-1H-pyrazole sulfate |
| **E15** | 1,4-diamino-2-methylbenzene sulfate |
| **E16** | 4-methylamino-phenol |

**Table 6:**

| **Couplers** | |
|---|---|
| **K11** | 1,3-diaminobenzene |
| **K12** | 2-amino-4-[(2-hydroxyethyl)amino]anisole-sulfate |
| **K13** | 2,4-diamino-1-(2-hydroxyethoxy)benzene sulfate |
| **K14** | 2,4-diamino-1-fluoro-5-methylbenzene sulfate |
| **K15** | 3-amino-6-methoxy-2-(methylamino)pyridine |
| **K16** | 3,5-diamino-2,6-dimethoxypyridine dihydrochloride |
| **K17** | 2,4-diamino-1-(2-hydroxyethoxy)-5-methylbenzene sulfate |
| **K18** | N-[(3-dimethylamino)phenyl]urea |
| **K19** | 1,3-di(2,4-diaminophenoxy)propane tetrahydrochloride |
| **K21** | 3-amino-phenol |
| **K22** | 5-amino-2-methylphenol |
| **K23** | 3-amino-2-chloro-6-methylphenol |
| **K24** | 5-amino-4-fluoro-2-methylphenol sulfate |
| **K25** | 1-naphthol |
| **K26** | 2-methyl-1-naphthol-acetate |
| **K31** | 1,3-dihydroxybenzene |
| **K32** | 1,3-dihydroxy-2-methyl-benzene |
| **K33** | 1-chloro-2,4-dihydroxybenzene |
| **K34** | 4-[(2-hydroxyethyl)amino]-1,2-methylenedioxybenzene*HCl |
| **K35** | 3,4-methylendioxy-phenol |
| **K36** | 1,5-dihydroxynaphthalene |
| **K37** | 2,7-dihydroxynaphthalene |
| **K38** | 5-[(2-hydroxyethyl)amino]-2-methyl-phenol |
| **K39** | 1,2,4-trihydroxybenzene |
| **K40** | 3-methyl-1-phenyl-5-pyrazolone |
| **K41** | 2-amino-6-methylphenol |
| **K42** | 2,6-dihydroxy-3,4-dimethylpyridine |
| **K43** | 2-amino-3-hydroxypyridine |
| **K44** | 3-amino-2,6-dimethylphenol |
| **K45** | 5-amino-2-ethylphenol |
| **K46** | 1,3-dihydroxy-2,4-dimethylbenzene |

**Table 7:**

| **Direct Dyes** | |
|---|---|
| **D1** | 2,6-diamino-3-[(pyridin-3-yl)azo]pyridine |
| **D2** | 6-chloro-2-ethylamino-4-nitro-phenol |
| **D3** | 2-amino-6-chloro-4-nitro-phenol |
| **D4** | 4-[(2-hydroxyethyt)amino]-3-nitro-1-methylbenzene |
| **D5** | 1-[2-ureidoethyl)amino]-4-nitrobenzene |
| **D6** | 4-[(2-hydroxyethyl)amino]-3-nitro-1-trifluoromethylbenzene |
| **D7** | 2,4-dinitro-6-((2-hydroxyethyl)amino)-phenol |
| **D8** | 1-amino-5-chloro-4-[(2,3-dihydroxypropyl)amino]-2-nitrobenzene |
| **D9** | 4-((2-hydroxyethyl)amino)-3-nitro-phenol |

**Table 8:**

| **Other Dyes** | |
|---|---|
| **W1** | 4-(2,5-diamino-benzylamino)-anilin*HCl |
| **W2** | 2-(3-amino-phenyl)aminomethyl-1,4-diamino-benzene*HCl |
| **W3** | 2-amino-5-ethylphenol phosphate |

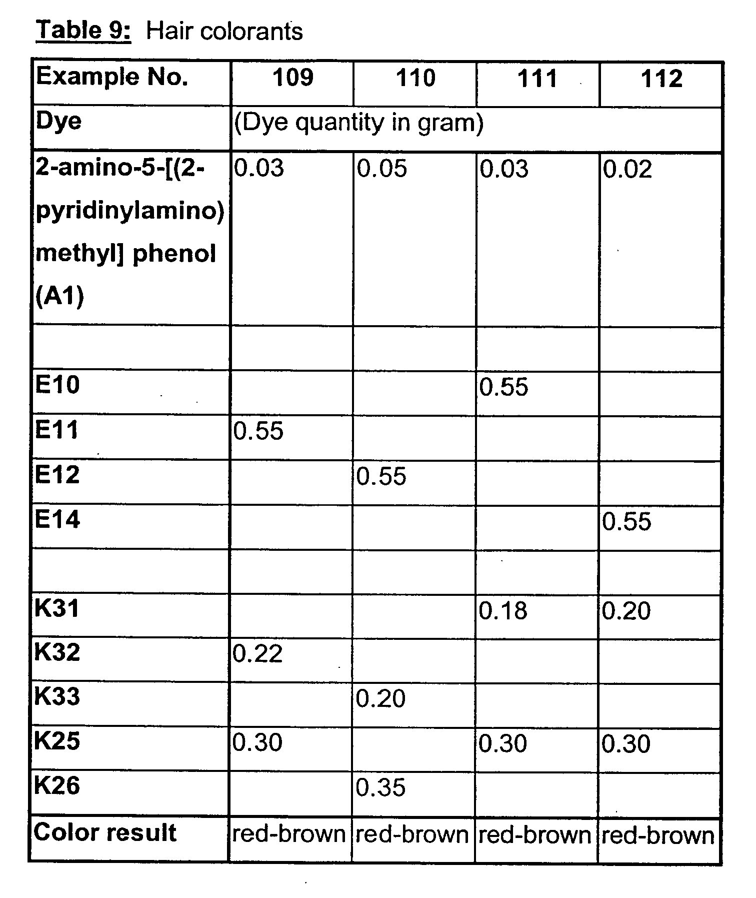

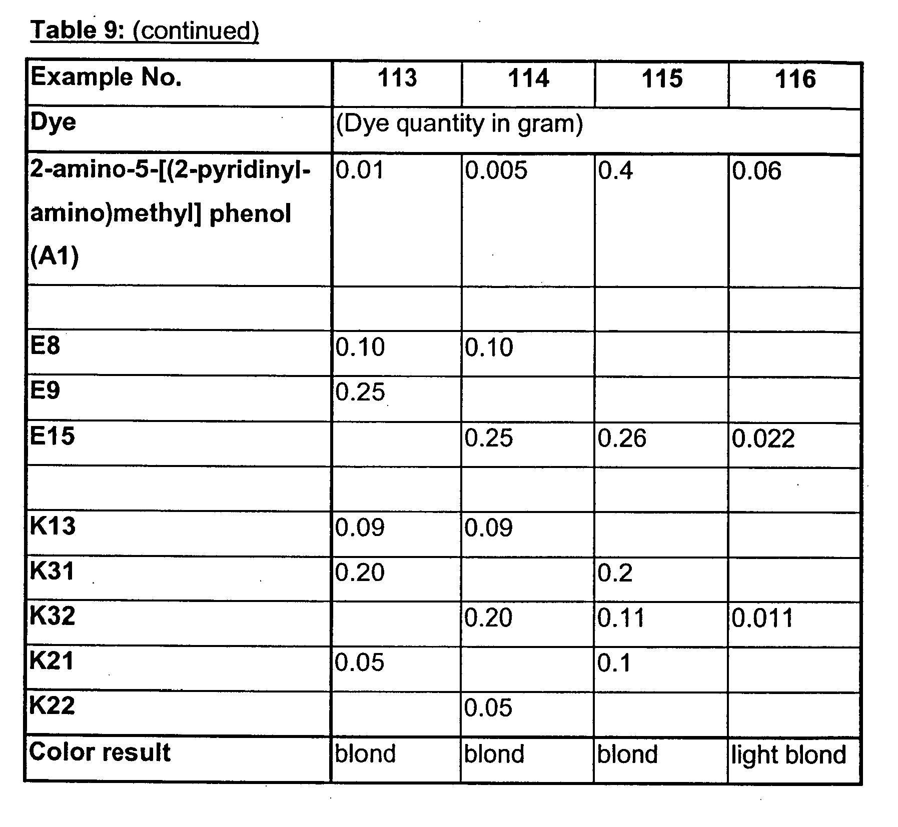

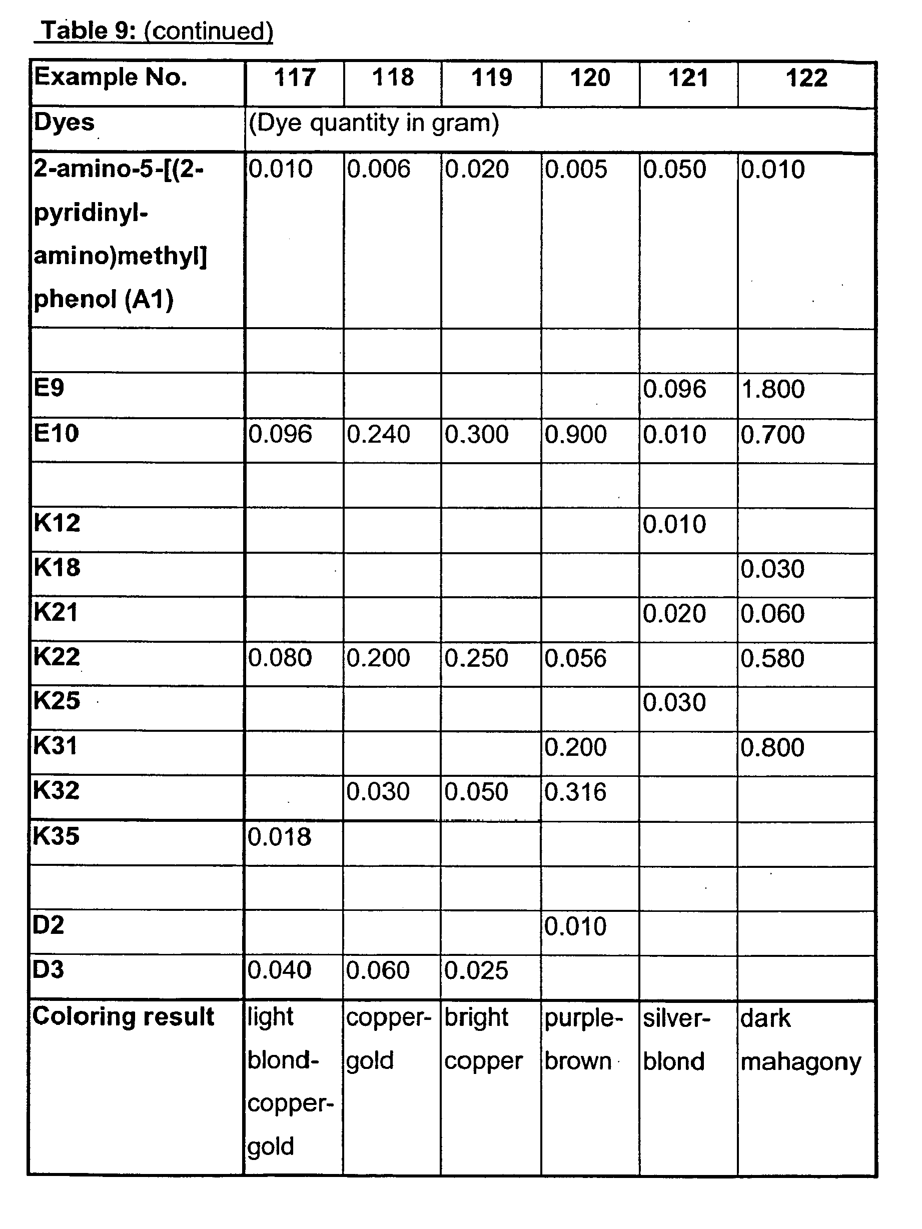

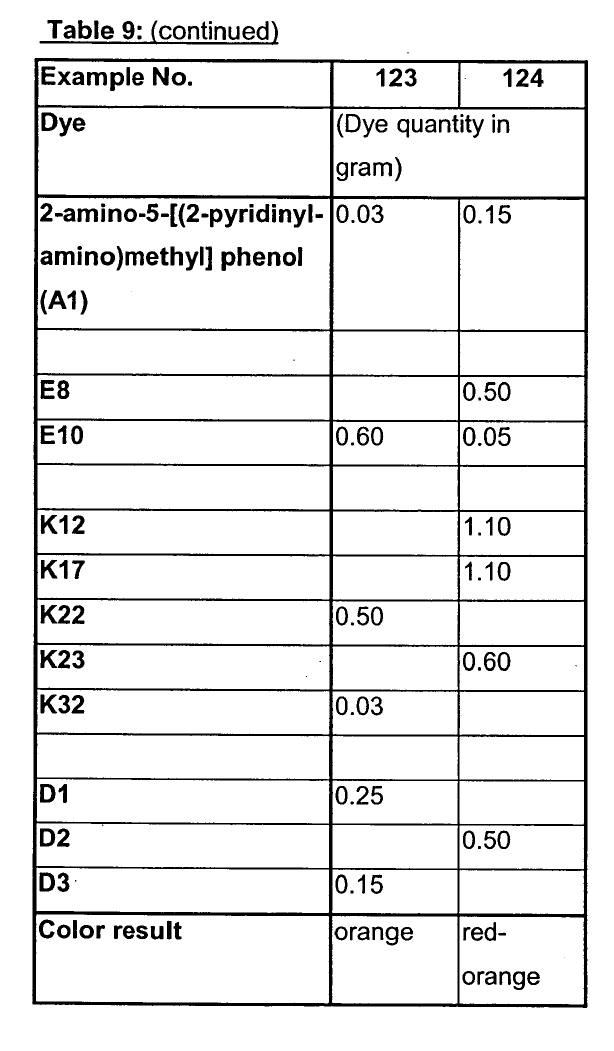

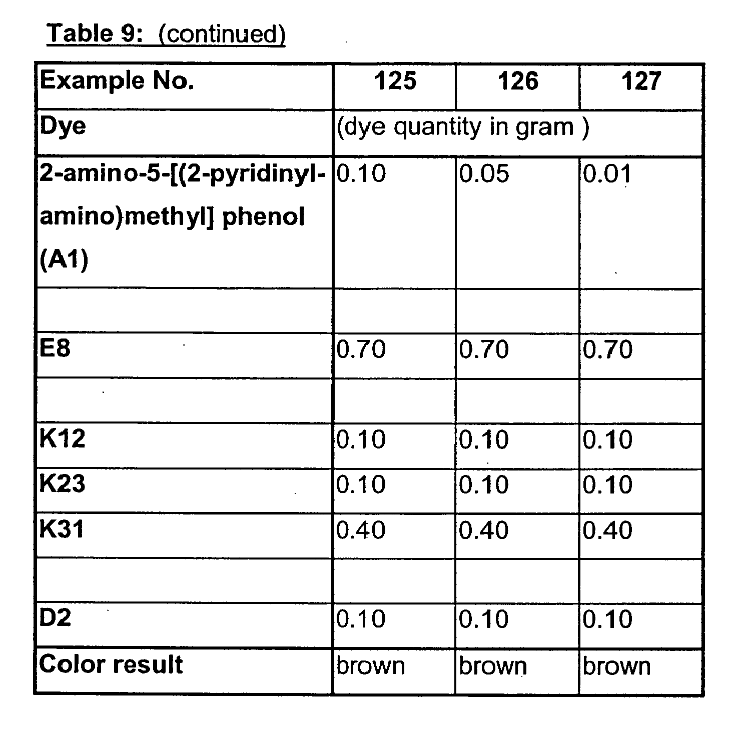

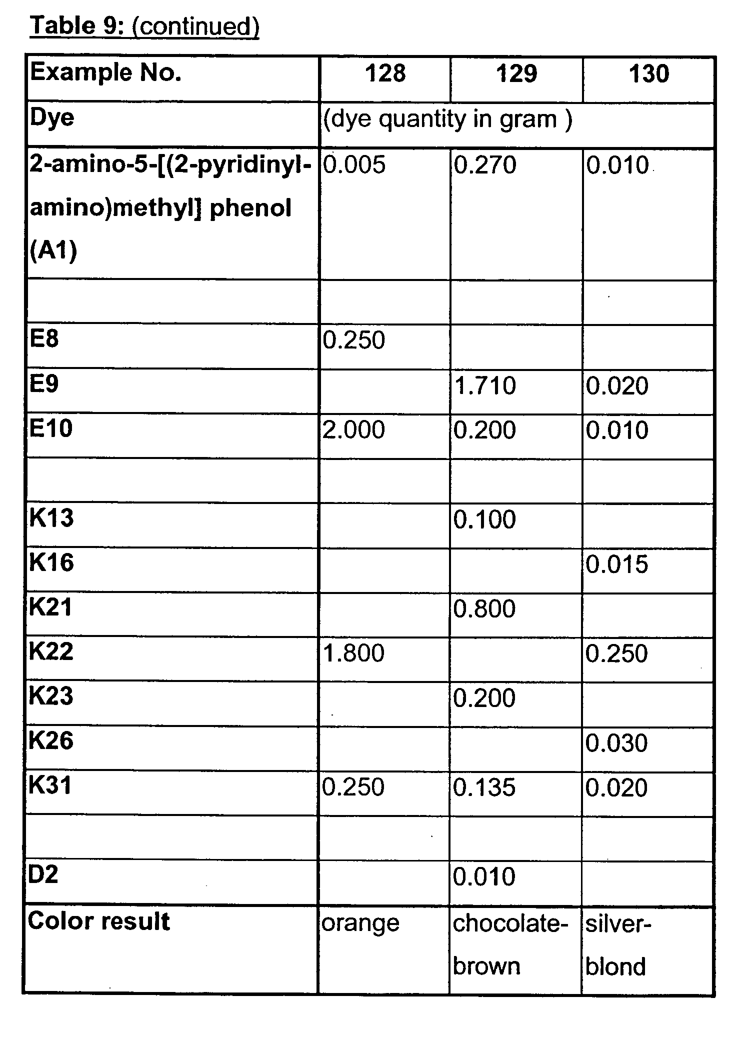

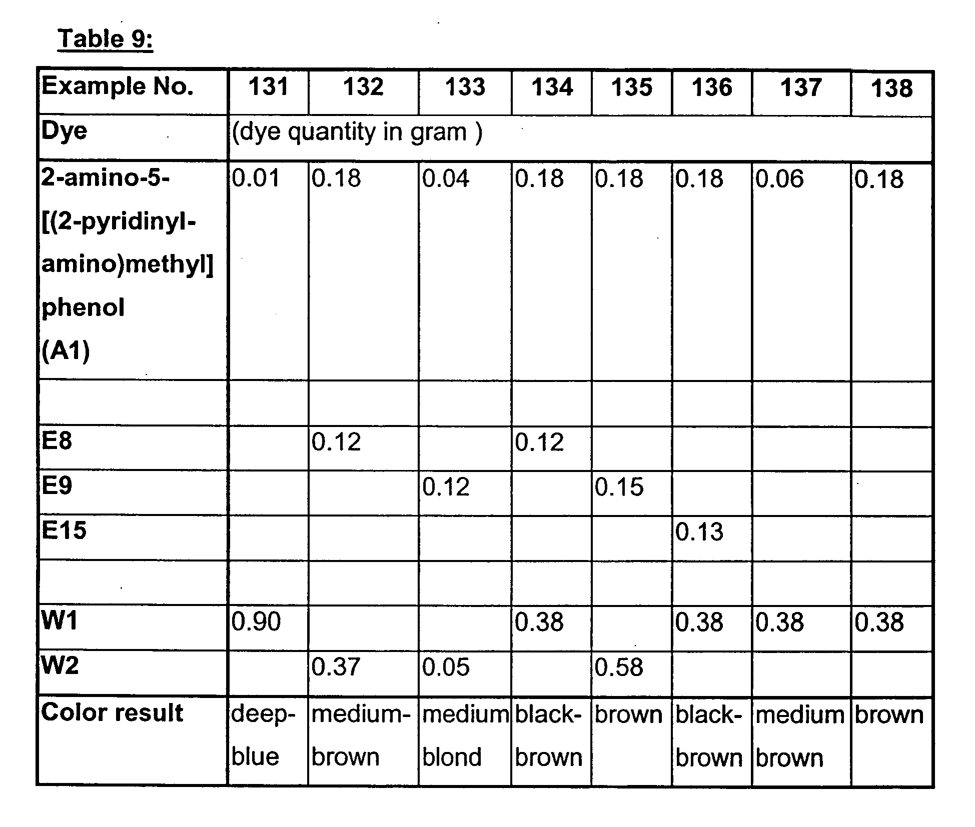

Unless otherwise indicated, all percentages are by weight.

## Claims

1. 2-amino-5-aminomethyl-phenol derivative of general of formula (I) 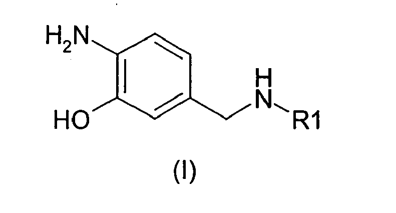 wherein **R1** denotes a five-membered or a six-membered heterocyclic ring, substituted or unsubstituted, or a physiologically tolerated, water-soluble salt thereof, with the proviso that R1 is not an unsubstituted 3-pyridinyl ring.

2. 2-Amino-5-aminomethyl-phenol derivative of general of formula (I) according to claim 1, **characterized in that** the 2-amino-5-aminomethyl-phenol derivative of general of formula (I) is selected from the group consisting of 2-amino-5-[(2-pyridinylamino)methyl]phenol, 2-amino-5-{[(3-methyl-2-pyridinyl)amino]methyl}phenol, 2-amino-5-{[(4-methyl-2-pyridinyl)amino]methyl}phenol, 2-amino-5-{[(5-methyl-2-pyridinyl)amino]methyl}phenol, 2-amino-5-{[(6-methyl-2-pyridinyl)amino]methyl}phenol, 2-amino-5-{[(4,6-dimethyl-2-pyridinyl)amino]methyl}phenol, 2-amino-5-{[(3-bromo-2-pyridinyl)amino]methyl}phenol, 2-amino-5-{[(4-bromo-2-pyridinyl)amino]methyl}phenol, 2-amino-5-{[(5-bromo-2-pyridinyl)amino]methyl}phenol, 2-amino-5-{[(3,5-dibromo-2-pyridinyl)amino]methyl}phenol, 2-amino-5-{[(3,6-dibromo-2-pyridinyl)amino]methyl}phenol, 2-amino-5-{[(6-bromo-5-methyl-2-pyridinyl)amino]methyl}phenol, 2-amino-5-{[(5-bromo-6-methyl-2-pyridinyl)amino]methyl}phenol, 2-amino-5-{[(3-chloro-2-pyridinyl)amino]methyl}phenol, 2-amino-5-{[(4-chloro-2-pyridinyl)amino]methyl}phenol, 2-amino-5-{[(5-chloro-2-pyridinyl)amino]methyl}phenol, 2-amino-5-{[(3,5-dichloro-2-pyridinyl)amino]methyl}phenol, 2-amino-5-{[(3,6-dichloro-2-pyridinyl)amino]methyl}phenol, 2-amino-5-{[(5-fluoro-2-pyridinyl)amino]methyl}phenol, 6-[(4-amino-3-hydroxybenzyl)amino]nicotinonitrile, 6-[(4-amino-3-hydroxybenzyl)amino]nicotinic acid, 6-[(4-amino-3-hydroxybenzyl)amino]-3-pyridinesulfonic acid, 2-amino-5-[2-chloro(3-pyridinylamino)methyl]phenol, 2-amino-5-[6-chloro(3-pyridinylamino)methyl]phenol, 2-amino-5-[2,6-dichloro(3-pyridinylamino)methyl]phenol, 5-[(4-amino-3-hydroxybenzyl)amino]-2-pyridinecarbonitrile, 2-amino-5-{[(6-methoxy-3-pyridinyl)amino]methyl}phenol, 2-amino-5-[(4-pyridinylamino)methyl]phenol, 2-amino-5-{[(2-methyl-4-pyridinyl)amino]methyl}phenol, 2-amino-5-{[(2-chloro-4-pyridinyl)amino]methyl}phenol, 2-amino-5-[(2-pyrimidinylamino)methyl]phenol, 2-amino-5-{[(4-methyl-2-pyrimidinyl)amino]methyl}phenol, 2-amino-5-{[(4-chloro-2-pyrimidinyl)amino]methyl}phenol, 2-amino-5-{[(4-bromo-2-pyrimidinyl)amino]methyl}phenol, 2-amino-5-{[(4-hydroxy-2-pyrimidinyl)amino]methyl}phenol, 2-amino-5-{[(4-methoxy-2-pyrimidinyl)amino]methyl}phenol, 2-amino-5-{[(5-methyl-2-pyrimidinyl)amino]methyl}phenol, 2-amino-5-{[(5-chloro-2-pyrimidinyl)amino]methyl}phenol, 2-amino-5-{[(5-bromo-2-pyrimidinyl)amino]methyl}phenol, 2-amino-5-{[(5-hydroxy-2-pyrimidinyl)amino]methyl}phenol, 2-amino-5-{[(5-methoxy-2-pyrimidinyl)amino]methyl}phenol, 2-[(4-amino-3-hydroxybenzyl)amino]-5-pyrimidinecarbonitrile, 2-amino-5-{[(4,6-dichloro-2-pyrimidinyl)amino]methyl}phenol, 2-amino-5-{[(4,6-dihydroxy-2-pyrimidinyl)amino]methyl}phenol, 2-amino-5-{[(4,6-dimethoxy-2-pyrimidinyl)amino]methyl}phenol, 2-amino-5-[(2-pyrazinylamino)methyl]phenol, 2-amino-5-{[(3-chloro-2-pyrazinyl)amino]methyl}phenol, 2-amino-5-{[(3-bromo-2-pyrazinyl)amino]methyl}phenol, 2-amino-5-{[(5-chloro-2-pyrazinyl)amino]methyl}phenol, 2-amino-5-{[(5-bromo-2-pyrazinyl)amino]methyl}phenol, 2-amino-5-{[(6-chloro-2-pyrazinyl)amino]methyl}phenol, 2-amino-5-{[(6-bromo-2-pyrazinyl)amino]methyl}phenol, 2-amino-5-[(1,3-thiazol-2-ylamino)methyl]phenol, 2-amino-5-{[(4-methyl-1,3-thiazol-2-yl)amino]methyl}phenol, 2-amino-5-{[(5-methyl-1,3-thiazol-2-yl)amino]methyl}phenol, 2-amino-5-{[(4,5-dimethyl-1,3-thiazol-2-yl)amino]methyl}phenol, 2-amino-5-[(5-isothiazolylamino)methyl]phenol, 2-amino-5-[(5-isoxazolylamino)methyl]phenol, 2-amino-5-[(1*H*-pyrazol-3-ylamino)methyl]phenol, 2-amino-5-[(1*H*-1,2,4-triazol-3-ylamino)methyl]phenol and 2-amino-5-[(1*H*-imidazol-2-ylamino)methyl]phenol or
their physiologically tolerated salts.

3. 2-Amino-5-aminomethyl-phenol derivative of general of formula (I) according to claim 1 or 2, **characterized in that** it is selected from the group consisting of 2-amino-5-[(2-pyridinylamino)methyl]phenol, 2-amino-5-{[(4-methyl-2-pyridinyl)amino]methyl}phenol, 2-amino-5-[(2-pyrimidinylamino)-methyl]phenol, 2-amino-5-[(2-pyrazinylamino)methyl]phenol, 2-amino-5-[(1,3-thiazol-2-ylamino)methyl]phenol, 2-amino-5-{[(4-methyl-1,3-thiazol-2-yl)amino]methyl}phenol, 2-amino-5-{[(5-methyl-1,3-thiazol-2-yl)amino]methyl}phenol, and 2-amino-5-{[(4,5-dimethyl-1,3-thiazol-2-yl)amino]methyl}phenol or their physiologically tolerated salts.

4. Coloring agent for the oxidative coloring of keratinic fibers, **characterized in that** it contains at least one 2-amino-5-aminomethyl-phenol derivatives of formula (I) according to any one of claims 1 to 3.

5. Coloring agent according to claim 4, **characterized in that** it contains the 2-amino-5-aminomethyl-phenol derivative of general of formula (I) in a total amount of from 0.005 to 20 % by weight.

6. Coloring agent according to claim 4 or 5, **characterized in that** it contains in addition to the 2-amino-5-aminomethyl-phenol derivative of general of formula (I) at least one additional developer or coupler.

7. Coloring agent according to any one of claims 4 to 6, **characterized in that** it contains the developers and couplers in a total amount of from 0.005 to 20.0 % by weight, based on the total amount of the oxidative coloring agent.

8. Coloring agent according to any one of claims 4 to 7, **characterized in that** it contains additionally at least one direct dye.

9. Coloring agent according to any one of claims 4 to 8, **characterized in that** it has a pH of from 6.8 to 11.5.

10. Coloring agent according to any one of claims 4 to 9, **characterized in that** it is in the form of an aqueous or aqueous-alcoholic solution, a cream, a gel or an emulsion.

11. Coloring agent according to any one of claims 4 to 10, **characterized in that** it is a hair colorant.
